# EUROPÄISCHE PATENTSCHRIFT

(11) **EP 1 152 706 B1**
(45) Veröffentlichungstag und Bekanntmachung des Hinweises auf die Patenterteilung: **23.03.2005**
(21) Anmeldenummer: 99955964.4
(22) Anmeldetag: 10.11.1999
(51) Int. Cl.: A61B 19/00

(54) **CHIRURGISCHES NAVIGATIONSSYSTEM MIT EINER MARKIERUNGSEINRICHTUNG UND EINEM ZEIGER FÜR EINE TRACKING EINRICHTUNG**
SURGICAL NAVIGATION SYSTEM HAVING A MARKING DEVICE AND A POINTER FOR A TRACKING DEVICE
SYSTEME DE NAVIGATION A USAGE CHIRURGICAL AVEC UN DISPOSITIF DE MARQUAGE ET UN POINTEUR POUR DISPOSITIF DE SUIVI

(30) Priorität: 17.11.1998 DE 19853010; 05.03.1999 DE 19909816
(43) Veröffentlichungstag der Anmeldung: 14.11.2001
(73) Patentinhaber: Schaerer Mayfield USA, Inc., Cincinnati, OH 45227 (US)
(72) Erfinder: HOELL, Thomas, D-06110 Halle/Saale (DE); WARSCHEWSKE, Udo, D-12249 Berlin (DE); VON STOCKHAUSEN, Hans-Martin, D-91054 Erlangen (DE)
(74) Vertreter: Schmidt, Steffen J., Dipl.-Ing.
(86) Internationale Anmeldenummer: PCT/EP1999/008602
(87) Internationale Veröffentlichungsnummer: WO 2000/028911

(56) Entgegenhaltungen:
- WO-A-95/09562
- WO-A-96/32059
- WO-A-97/29710
- WO-A-97/29803
- US-A- 5 617 857
- US-A- 5 776 064

## Beschreibung

Die Erfindung betrifft ein Navigationssystem nach dem Oberbegriff des Patentanspruchs 1 zur Durchführung und Unterstützung von chirurgischen Eingriffen, insbesondere auf dem Gebiet der Neurochirurgie und der Hals-Nasen-Ohrenheilkunde.

Neuro-Navigationssysteme dienen der Unterstützung bei der Durchführung chirurgischer Eingriffe am Schadet. Bekannte Navigationssysteme ermöglichen es, die Position eines Operationsinstruments im Operationsfeld anzuzeigen, wobei kleine und tiefsitzende Läsionen im Gehirn auf atraumatische Weise zielsicher aufgesucht werden können. Bei operativen Eingriffen auf dem Gebiet der Hals-Nasen-Ohrenheilkunde kann mit Hilfe eines Navigationssystems beispielsweise sicher zwischen den Grenzen der Nebenhöhlen und dem Gehirn unterschieden werden.

Navigationssysteme, die mit unterschiedlichen Tracking- Verfahren arbeiten, sind bekannt. Beispielsweise soll hier auf das StealthStation-System der Firma Sofamor Danek Inc, USA, verwiesen werden. Bei den bekannten Systemen wird zunächst präoperativ eine Bildaufnahme von der Anatomie bzw. dem Gehim des Probanden bzw. Patienten angefertigt. Über ein Tracking- System mit optischem Sensor besteht dann die Möglichkeit, unter Nutzung der präoperativ gewonnenen Bilddaten auf einem Monitor Schnitte oder Ansichten des Gehirns darzustellen. Beim präoperativen Scannen mittels Computer- und/oder Kernspintomographie werden zur Koordinatenzuordnung sogenannte Fiducial-Marker auf der Schädenoberfläche des Patienten befestigt. Diese Fiducials dienen zum Lokalisieren der Aufnahmen unter Beachtung der jeweiligen räumlichen Orientierung.

Der Einsatz bekannt er Systeme wird durch einen aufwendigen Geräteaufbau sowie durch eine komplizierte und zeitaufwendige Bedienerführung, insbesondere bei optischem Tracking eingeschränkt.

Ebenfalls bekannte Tracking-Verfahren, welche auf die Erfassung der Position eines Zeigers für das bilddarstellende System auf magnetische Wechselfelder zurückgreifen, sind in einer Umgebung, die zu magnetischen Störeinflüssen führt, wie dies im klinischen Bereich regelmässig der Fall ist, wenig geeignet.

Weitere Nachteile bekannter Systeme bestehen in der komplizierten Handhabung, da der Operateur zusätzlich zum Zeiger eine Computertastatur bedienen muss, um die Bilddarstellung auszulösen oder Steuerungs- oder Markierungsoperationen auszuführen.

In dem Fall, wenn optische Sensoren bzw. optisches Tracking zur Anwendung kommt, sind aufwendige Kalibrierungsmassnahmen notwendig, insbesondere dann, wenn der Operationstisch mit dem Patienten im Raum bewegt wird und sich hierdurch unerwünschte Lageveränderungen der optischen Sensorik einstellen. Zur Überwindung dieser Problematik wird auf dem Schädel, bzw. an der Kopfhalterung, welche fest mit dem Schädel verbunden ist, ein optisches Referenzierungssystem angebracht. Dieses überwindet zwar das Kalibrierungsproblem, aufgrund der Grösse dieser Referenzierungsmarker und ihrer Anfälligkeit gegen mechanische Veränderung sind sie beim Operieren jedoch störend.

Bisher bekannte Fiducials, d. h. Markierungseinrichtungen zum Erstellen von Bilddaten mittels Kernspin- und/oder Computertomographie bzw. zur Positionserfassung der Lage eines Probanden und Koordinatenzuordnung bei chirurgischen Eingriffen mit Unterstützung eines Navigationssystems besitzen eine relativ grosse räumliche Ausdehnung und führen zu unangenehmen Behinderungen des Patienten, insbesondere bedingt durch den zeitlichen Abstand zwischen den Kernspin- und Computertomographie-Aufnahmen einerseits und dem späteren operativen Eingriff andererseits. Zur Koordinatenzuordnung müssen nämlich die Fiducials am Kopf des Patienten verbleiben, was augenscheinlich unangenehm ist.

Die für eine Tracking-Einrichtung eines Navigationssystems notwendigen Zeiger, auch mit Stylus oder Pen bezeichnet, müssen den Anforderungen hinsichtlich des klinischen Einsatzes einschliesslich der Sterilisierbarkeit, insbesondere der Dampfsterilisat genügen. Gleichzeitig sollen derartige Zeiger leicht und einfach zu handhaben sein und über entsprechende Mittel zum Auslösen von Schalt- oder Steuerbefehlen verfügen.

Aus der WO 96/32 059 ist ein Navigationssystem gemäß dem Oberbegriff des Anspruchs 1 sowie ein Zeiger bekannt. Diesem Stand der Technik ist keine Information darüber zu entnehmen, wie das offenbarte Navigationssystem handzuhaben und zu bedienen ist.

Aus dem Vorgenannten ist es Aufgabe der Erfindung, ein komplexes Navigationssystem zur Durchführung und Unterstützung von chirurgischen Eingriffen anzugeben, wobei das System auf eine Tracking-Sensorik zurückgreifen soll, die eine hochgenaue Positionserfassung ermöglicht, ohne dass das Operationsfeld eingeschränkt bzw. die Tätigkeit des Operateurs behindert wird. Weiterhin soll das Navigationssystem nahezu in Echtzeit aus präoperativen Aufnahmen respektive einer Bilddatenbank eine 3D-Visualisierung entsprechend der gewünschten Blickrichtung, die mit Hilfe eines Zeigers definiert wird, ermöglichen. Mit dem zu schaffenden Navigationssystem soll auch eine Einhand-Steuerung der notwendigen Vorgänge zur Visualisierung, Speicherung und so weiter realisierbar sein.

Die Lösung der Aufgabe der Erfindung erfolgt hinsichtlich des Navigationssystems gemäß der Definition nach der Lehre des Patentanspruchs 1.

Die Unteransprüche stehen hierbei mindestens zweckmässige Ausgestaltungen und Weiterbildungen des jeweiligen Erfindungsgegenstands bzw. des Systems dar.

Der erfindungsgemässe Grundgedanke bezüglich des Navigationssystems zur Durchführung und Unterstützung von chirurgischen Eingriffen besteht in der Fortbildung bekannter Lösungen dergestalt, dass für die Tracking-Einrichtung zum Bestimmen der momentanen Position eines Instruments und Ableiten oder Realtime-Nachziehen von Darstellungen der Patientenanatomie anhand der in einer Bilddatenbank abgelegten Aufnahmen auf Mittel zum Erzeugen eines definierten magnetischen Gleichfelds in der Navigationsumgebung zurückgegriffen wird, wobei ein Zeiger-navigationsinstrument zur Anwendung kommt, welches einen integralen Magnetfeldsensor, abgestimmt auf das magnetische Gleichfeld des Transmitters, aufweist.

Erfindungsgemäß wird weiterhin durch ein Softwaremodul ein Extrahieren von anatomischen Strukturen aus den Rohdatensätzen der präoperativen Aufnahmen und Bereitstellen dieser Strukturen in Form von visualisierbaren 3D-Datensätzen möglich.

Durch das erwähnte Bildbearbeitungsmodul können anatomische Strukturen eigenschafts- oder umgebungsorientiert aus den Eingangs-Summendatensätzen, die präoperativ gewonnen wurden, selektiert werden und auf der Basis einer vorgebbaren Segmentierungsstrategie in diskrete Datensätze umgewandelt werden.

Ein weiterer Grundgedanke des erfindungsgemäßen Navigationssystems besteht darin, daß Mittel zur Anwendung kommen, welche eine Menü-geführte Steuerung des Systems durch Bewegungen des Zeigernavigationsinstruments außerhalb des Operationsfelds, aber innerhalb einer Navigationsumgebung gestatten, indem ein Aktivieren oder Deaktivieren angebotener Menüs oder Steueraktivitäten allein durch die vorerwähnte Bewegung des Navigationsinstruments vornehmbar ist.

Der spezielle Gleichfeld-Transmitter zum Erzeugen eines Magnetfelds für die Tracking-Einrichtung ist am Operationstisch oder einer dort vorgesehenen Kopfauflage, in einer festen Verbindung zum Probanden stehend, jedoch außerhalb des Operationsfelds und damit nicht störend angeordnet. Hierdurch ergibt sich eine feste, reproduzierbare Lagebeziehung zwischen dem Probanden, welcher am bzw. auf dem Operationstisch, insbesondere einer Kopfauflage fixiert ist, und dem Transmitter, unabhängig von der Lage des Operationstisches im Raum selbst.

Der Magnetfeldsensor stellt Signale zum Ableiten von Position und/oder Bewegungsrichtung des Zeigernavigationsinstruments anhand des definierten magnetischen Gleichfelds und dessen Orientierung bereit, wobei diese Signale sowohl auf einem Monitor darstellbar sind als auch zur Steuerung der Nachladeund Aktualisierungsvorgänge des Bildbearbeitungsmoduls Anwendung finden.

In einer bevorzugten Ausgestaltung der Erfindung ist ein weiterer Magnetfeldsensor vorgesehen, wobei dieser Sensor am Probanden, vorzugsweise cranial, d.h. am Schädel oder einem anderen zu navigierenden Körperteil befestigbar ist. Hierdurch können Lage- und Positionsveränderungen des Schädels oder Körperteils bezogen auf den Gleichfeld-Transmitter erfaßt werden. Durch diesen zusätzlichen Sensor können Lageabweichungen, die ansonsten zu einem neuen Einmessen oder Einjustieren des Systems führen, systemintern automatisch korreliert bzw. ausgeglichen werden. Der Operateur kann in diesem Fall, wenn dies operationstechnisch erforderlich ist, die Position des Schädels in gewünschter Weise verändern, ohne daß die Navigationsmöglichkeit durch fehlende Justierung oder erforderliches Neujustieren eingeschränkt ist. Mit anderen Worten ermöglicht der weitere Magnetfeldsensor das Bereitstellen von Korrekturdaten, die dem Steuerrechner zugeführt werden, um ein quasi dynamisches oder bezogen auf die Ursprungsänderung veränderliches Koordinatensystem zu bestimmen.

Mittels des vorstehend kurz beschriebenen Systems wird der Operateur respektive der Bediener weitgehend entlastet und die Zahl möglicher Fehlerquellen in der Bedienerführung reduziert. Dies ist insbesondere durch das virtuelle Steuerpult möglich, mit Hilfe dessen der Operateur ohne fremde Hilfe alle wesentlichen Funktionen des Navigationssystems bzw. des dort implementierten Computers bedienen und kontrollieren kann. Im einzelnen besteht die Möglichkeit, die Darstellungsformen zu ändern, die Ansichten der anatomischen Strukturen und/oder das Einschalten spezifischer Funktionen vorzunehmen. Hierfür wird ein übersichtliches Menü auf dem Monitor oder Display genutzt, wobei ein Aktivieren oder Deaktivieren durch die erwähnte einfache räumliche Bewegung des Navigationsinstruments außerhalb des Operationsfelds realisiert wird.

Die Anwendung eines Tracking-Systems auf der Grundlage von Feldstärkemessungen von konstanten, d.h. Gleichmagnetfeldern, wird eine Signalverfälschung, verursacht durch elektromagnetisch induzierte Feldüberlagerung, wirksam unterdrückt. Die Komponenten des Navigationssystems, insbesondere der Gleichfeld-Transmitter, können unterhalb steriler Operationsfeld-Abdeckungen angeordnet werden, wodurch das Umfeld und Sichtfeld des Operateurs nicht eingeschränkt ist und eine Störung des operativen Ablaufs entfällt. Im Vergleich zu optischen Navigationssystemen ist der Aufbau der erfindungsgemäßen Lösung unempfindlich gegenüber mechanischen Erschütterungen. Zusätzlich kann der Patient ohne Navigationseinschränkung frei mit dem Operationstisch im Raum bewegt werden. Die automatisierte Bildbearbeitung des vorliegenden Systems führt zu einer wesentlichen Verkürzung der Vorbereitungszeiten, wobei die 3D-Darstellung des Gehirns auf dem Monitor und der Anblick des Gehirns nach Craniotomie identisch sind, wodurch der Chirurg bereits vor dem Eingriff erweiterte Möglichkeiten zur Operationsplanung erhält.

Für die Anwendung des Navigationssystems wird in an sich bekannter Weise der Kopf des Patienten vor der Operation durch den Neurochirurgen fixiert, wobei auf entsprechende mechanische Kopfstützen zurückgegriffen wird. Das Kopfstützsystem kann aus gängigen Materialien wie Aluminium oder hochlegiertem Stahl bestehen, ohne daß Störungen der Tracking-Einrichtung zu befürchten sind. Mit Hilfe des Navigationssystems wird beim Einmessen die Kopfposition des Patienten im Raum registriert und es wird zunächst automatisch eine Bildperspektive aus der Sicht des Operateurs in einer 3D-Grafikdarstellung angeboten. Die Position des Operationstisches selbst hat keinen Einfluß auf den Navigationsprozeß, weil eine feste Lagebeziehung zwischen der Kopfhalterung und dem Transmitter der Tracking-Einrichtung besteht.

Für bevorzugte neurochirurgische Anwendung wird, wie dargelegt, zunächst das 3D-Abbild des Gehirns zu Beginn des Eingriffs aus der Blickperspektive des Operateurs dargestellt. Diese Perspektive kann jedoch unter Verwendung des Navigationsinstruments, d.h. des Zeigers, welcher später beschrieben wird, jederzeit geändert werden.

In einer Ausführungsform der Erfindung ermöglicht das Navigationssystem als Zusatzfunktion die Dokumentation elektrophysiologischer Messungen. Hierfür kann auf die Bildschirmdarstellung der Hirnoberfläche das Auftragen von Markierungen erfolgen, die mit Hilfe des Navigationsinstruments, d.h. des Zeigers gesetzt werden. Mit dem Zeiger/Stylus sind Papier-Markierungen, sogenannte Tags, anfahrbar und über Knopfdruck können diese Positionen gespeichert werden. Der Operateur kann Ziffern und Farben über ein am Monitor dargestelltes Steuerfeld auswählen. Zusätzlich besteht die Möglichkeit, die Dokumentation mit einer Zeitmarkierung zu ergänzen. Die so erstellten Dokumente sind abspeicherbar und können zu einem späteren Zeitpunkt über einen angeschlossenen Drucker ausgegeben werden, was für die nachfolgende Operationsbeschreibung und -abrechnung von Vorteil ist.

Für die Navigation ist es, wie allgemein bekannt, notwendig, eine Verbindung zwischen dem virtuellen Bild des Gehirns und dem realen Gehirn des im Operationssaal auf dem Operationstisch befindlichen Patienten herzustellen. Hierfür werden eindeutige Positionen benötigt, die sich sowohl im realen als auch im virtuellen System wiederfinden. Grundsätzlich kommen anatomische Landmarken zur Anwendung, wobei durch Verschiebungen der Haut und die Größe der Landmarken eine punktgenaue Zuordnung nicht immer erreicht werden kann.

Aus vorstehend genanntem Grund weisen bekannte Navigationssysteme zusätzlich Referenzpunkte bzw. Marker oder Fiducials auf. Diese Marker bestehen aus Materialien, die sich bei der Kernspin- und/oder Computertomographie nachweisen lassen.

Unter Beachtung der erforderlichen Präzision, aber auch bei Berücksichtigung der Hautverschiebung wurden Markersysteme bekannt, die durch Verschraubung am Schädelknochen fixiert werden. Alternativen bestehen in dem Einsatz von Gebißschienen, die der Patient während der Untersuchung bei der Tomographie und auch im Operationssaal fest im Mund halten muß. Derartige Methoden sind mit einem hohen finanziellen und zeitlichen Aufwand verbunden und in ihrer Anwendung für den Patienten äußerst unangenehm. Das Einschrauben von Markern kann aufgrund der hiermit verbundenen invasiven Handlung im Regelfall nur von einem Arzt durchgeführt werden, was die Kosten weiter erhöht.

Gemäß einem ergänzenden Grundgedanken der Erfindung wurde daher eine spezielle Markierungseinrichtung bzw. Fiducial zum Erstellen von Bilddaten für eine Datenbank mittels Tomographie einerseits sowie andererseits zur Positionserfassung der Lage eines Probanden und Koordinatenzuordnung beim chirurgischen Eingriff geschaffen.

Erfindungsgemäß ist ein Träger vorhanden, welcher als flächiges, mindestens teilflexibles Gebilde mit einem im wesentlichen zentrischen, auf der der Klebefläche abgewandten Seite hervorstehenden Rastknopf vorliegt.

Die eigentliche Markierungssubstanz wird von einem im wesentlichen zylindrischen Gehäuse, welches leicht handhabbar ist, aufgenommen. Der Gehäuseboden weist bevorzugt eine konkave Form oder einen Rücksprung auf, wobei am Gehäuseboden eine im wesentlichen zentrische Rastaufnahme oder bezogen auf den Rastknopf ein Rastgegenstück angeordnet ist.

Der Rastknopf einerseits und die Rastaufnahme oder das Rastgegenstück andererseits ermöglichen das Anbringen des zylindrischen Gehäuses nach dem Befestigen des Trägers am Probanden respektive das Lösen des Gehäuses zu einem späteren Zeitpunkt einschließlich Wiederanbringen des letzteren.
Der Patient kann so nach oder zwischen den Tomographie-Aufnahmen vom relativ großvolumigen zylindrischen Gehäuse mit Markierungssubstanz befreit werden und sich ungehindert bewegen. Für weitere Aufnahmen und zur späteren operativen Behandlung sind die zylindrischen Gehäuse positionsgenau wieder befestigbar oder es können spezielle Einmeßhilfen im Operationssaal unter Nutzung des Rastknopfs aufgesteckt werden.

Die gewählte Klebebeschichtung in Verbindung mit der konkaven oder einen Rücksprung aufweisenden Form des Gehäusebodens, wobei die Krümmung in etwa der Schädelkalotte entspricht, stellt sicher, daß der folienartige Träger bzw. die Klebeplatte zum Kunststoffkörper, d.h. zum Gehäuse für die Markierungssubstanz herangezogen wird. Auf diese Weise gelingt es, auch bei verschiedenen Krümmungsradien, d.h. unterschiedlichen Schädelabschnitten einen gleichbleibenden Abstand zwischen Markierungssubstanz, die sich beispielsweise in einem Kugelgehäuse befindet, und der Hautoberfläche zu erreichen. Letztendlich werden hierdurch auch Fehler beim Einmessen vermieden, da der Einmeßkörper von den Abmessungen her, insbesondere bezüglich der konkaven oder Rücksprung-Ausbildung des Bodens dem zylindrischen Körper mit Markierungssubstanz oder einer Aufnahme hierfür entspricht.

In einer Ausführungsform weist das zylindrische Gehäuse der Markierungseinrichtung einen abnehmbaren Gehäusedeckel, vorzugsweise aus einem durchsichtigen Kunststoff auf, so daß die Markierungssubstanz kontrolliert werden kann, aber auch austauschbar ist.

Die Markierungssubstanz für die Kernspintomographie selbst ist eine Flüssigkeit oder ein Gel, welche bzw. welches sich in einem Kugel- oder geschlossenen Zylindergefäß befindet, wobei der Außendurchmesser der Kugel oder des Zylinders im wesentlichen dem Innendurchmesser des zylindrischen Gehäuses mit Rastaufnahme oder Rastgegenstück entspricht. Die verwendete Substanz ist so gewählt, daß sie im Kernspin sowie im T1- als auch im T2-gewichteten Bild gut sichtbar ist. Die Besonderheit im Vergleich zu bekannten Markern liegt darin, daß diese üblicherweise nur im T1- oder im T2-Bild gut sichtbar sind. Gleichzeitig ist die Markierung über den Kontrastunterschied zwischen der Kugel und dem Gehäuse auch in der Computertomographie gut zu erkennen. Auf diese Art kann selbst bei der Wahl der falschen Markierungshilfe noch relativ problemlos jede beliebige, vom Benutzer ausgewählte Markierung auf den CT- oder Röntgenbildern erkannt werden. Diese Maßnahme dient dazu, im Falle einer falsch ausgewählten Markierungshilfe den Patienten nicht nochmals einmessen zu müssen und insbesondere bei der CT-Markierung eine zusätzliche Strahlenbelastung zu verhindern.

Es sei an dieser Stelle angemerkt, daß selbstverständlich auch eine kinematische Umkehr von Rastknopf und Rastaufnahme oder Rastgegenstück denkbar ist, ohne das erfindungsgemäße Prinzip der Trennung von Träger und zylindrischem Körper zur Aufnahme der eigentlichen Markierungssubstanz zu verlassen.

Wie bereits kurz dargelegt, ist erfindungsgemäß ein Einmeßkörper vorgesehen, welcher ein geschlossenes, in seinen Abmessungen und seiner bodenseitigen Ausbildung dem zylindrischen Gehäuse entsprechende Gestalt besitzt. Der Einmeßkörper weist deckelseitig eine Markierungsausnehmung auf, welche sich in einer solchen Position befindet, die gleich dem Mittel- oder Schwerpunkt der Kontrastmarkierungssubstanz bzw. des Kugel- oder Zylindergefäßes für eine derartige Substanz ist. Diese Markierungsausnehmung kann mit einem Zeiger oder Stylus angetastet werden, um den Einmeßvorgang zu erleichtern.

Bevorzugt besteht das zylindrische Gehäuse zur Aufnahme der Markierungssubstanz und/oder der Einmeßkörper aus Kunststoffmaterial, wobei der Träger bevorzugt eine einseitige im Bereich des Rastknopfs verstärkte klebebeschichtete Folie umfaßt.

Speziell zur Anwendung für das beschriebene Navigationssystem wird ein Zeiger für die erforderliche Tracking-Einrichtung vorgeschlagen, wobei der Zeiger die Form eines langgestreckten Handstück-Gehäuses besitzt, weiterhin im Gehäuseinneren ein vorzugsweise gekapselter Sensor vorhanden ist und mindestens teilweise aus dem Gehäuse eine Tastspitze oder eine Tastspitzen- bzw. Einführhilfeaufnahme hervorsteht.

Erfindungsgemäß ist der gekapselte Sensor mit der Tastspitze oder der Tastspitzenaufnahme mittels eines gegenüberliegende Öffnungen aufweisenden Körpers starr verbunden.

Der Verbindungskörper mit jeweils in den Öffnungen befindlichem Sensor und Tastspitze ist dann bezüglich des Handstück-Gehäuses nachgiebig, d.h. spannungsfrei, quasi kardanisch gelagert.

Der Verbindungskörper wird aus einem verformungsstabilen und temperaturbeständigen Kunststoff oder Titan hergestellt. Um die gewünschte Spannungsfreiheit zwischen Verbindungskörper und damit der Verbundanordnung aus Sensor und Tastspitze bezüglich des Gehäuses zu erreichen, ist zwischen verbindungskörper und der Gehäuseinnenseite ein Ringspalt ausgebildet. Zur Abdichtung zwischen Verbindungskörper und Gehäuse oder einem Überwurf und dem Gehäuse sind elastische Dichtmittel, bevorzugt elastische Dichtringe vorhanden.

Mit Hilfe des Zeigers, d.h. der dort vorhandenen Tastspitze, berührt der Operateur den Teil des Situs, der zu identifizieren und mit Hilfe der Navigationseinrichtung darzustellen ist. Der im Handstück befindliche Sensor übermittelt die Position des Zeigers und damit auch die Position und Richtung der Spitze und leitet diese zum Navigationsgerät weiter. Das Navigationsgerät ermöglicht dann eine Darstellung quasi von der Position der Spitze und der gegebenen Ausrichtung im Raum ausgehend auf dem Display bzw. Monitor. Hierfür wird durch eine geeignete Kabelverbindung oder auf drahtlosem Wege eine Übermittlung der Positionswerte in ein virtuelles Abbild des realen Situs vorgenommen.

Der Zeiger dient demnach der Bedienung des gesamten Systems, wobei hier, wie eingangs erwähnt, zwischen einem Operationsfeld und einem Steuerfeld unterschieden wird. In dem Fall, wenn der Operateur den Zeiger in das Steuerfeld hinein bewegt, wird die Auswahl und das Aktivieren von auf dem Monitor angebotenen Menüs und damit die Steuerung des Systems möglich.

Das Bestätigen oder Auslösen einer angewählten Funktion kann über einen im Zeiger angeordneten Taster, der über eine Signalleitung mit dem Navigationssystem bzw. dem Steuerrechner in Verbindung steht, erfolgen.

Die Tastspitze des Zeigers besteht aus einem biegefesten Material, vorzugsweise Edelstahl oder Titan. Alternativ besteht über die erwähnte Aufnahme die Möglichkeit, Punktionshilfen oder Einführhilfen für Kateder und so weiter zu befestigen. Den eingesetzten Spitzen ist gemeinsam, daß sie einen zum untersuchenden Objekt gerichteten Punkt aufweisen, der als Navigationsbezugspunkt dient. Der Bezugspunkt selbst ist frei wählbar. Letzteres ist möglich, weil der im Zeiger befindliche Sensor des Navigationssystems unabhängig vom System selbst auf das gegebene virtuelle Zentrum mißt. Die Spitze des Zeigers wird über einen Vektor definiert, der zur gegebenen Position des Sensors hinzugerechnet wird. Zur Bestimmung dieses Vektors ist die bereits erwähnte Einmeßprozedur notwendig. Konkret wird der Zeiger an einer festen Position, z.B. unter Verwendung der Einmeßhilfe um seine Spitze bewegt. Das Navigationssystem erfaßt dabei mit Hilfe der Tracking-Einrichtung die Bewegung und berechnet aufgrund der bekannten Position der Spitze, wie sich die Spitze des Systems zur Lage des Sensors verhält.

Der erfindungsgemäße Zeiger für die Tracking-Einrichtung des Navigationssystems ist weitgehend flüssigkeits- und dampfdicht, so daß eine Sterilisation, wie klinisch üblich, möglich ist. Durch den Verbindungskörper und die Anordnung des letzteren im langgestreckten Gehäuse ist gewährleistet, daß auch bei Spannung und/oder Temperaturwechselbelastungen die Position zwischen Sensor und Spitze unverändert bleibt, um die geforderten Genauigkeiten bei der Positionserfassung und Navigation zu erreichen.

Die Erfindung soll nachstehend anhand von Ausführungsbeispielen sowie unter Zuhilfenahme von Figuren näher erläutert werden.

Hierbei zeigen:
- Fig. 1: einen prinzipiellen Ablauf eines bildgestützten Navigationsverfahrens;
- Fig. 2: die Anordnung eines Transmitters der Tracking-Einrichtung am Operationstisch;
- Fig. 3: die Anordnung eines weiteren Sensors zur Detektion der Kopfbewegung des Patienten;
- Fig. 4: die Aufteilung von Operationsfeld und virtuellem Steuerfeld zur Bedienung des Systems mittels Zeiger bzw. Stylus;
- Fig. 5: eine Monitordarstellung mit dem Menü für die Markerregistrierung;
- Fig. 6: eine Monitordarstellung mit dem Menü Überprüfung der Marker bzw. Fiducials;
- Fig. 7: eine Monitordarstellung mit virtuellem Keypad;
- Fig. 8: eine Darstellung mit dem Feld zum Auslösen und Durchführen intraoperativer Dokumentationen;
- Fig. 9: eine Schnittdarstellung der teilbaren Markierungseinrichtung;
- Fig. 10: eine perspektivische Darstellung der kompletten Markierungseinrichtung;
- Fig. 11: eine perspektivische Darstellung des Trägers der Markierungseinrichtung, jedoch mit aufgestecktem Einmeßkörper; und
- Fig. 12: eine Schnittdarstellung des Zeigers oder Stylus für die Tracking-Einrichtung.

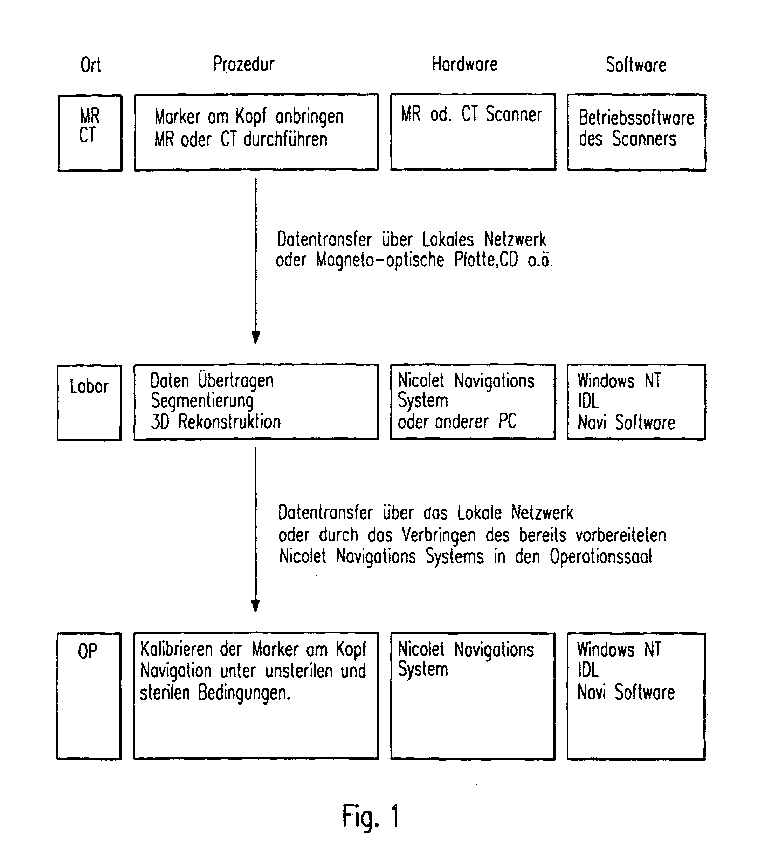

Das Navigationssystem gemäß Ausführungsbeispiel benötigt möglichst genaue Bilddaten der anatomischen Strukturen des Patienten. Diese Bilddaten werden, wie in der Fig. 1 gezeigt, entweder durch Computertomographie und/oder Kernspintomographie bereitgestellt.

Computertomographische Darstellungen sind dann vorteilhaft, wenn es sich um Abbildung von knöchernen Strukturen handelt. Für die Darstellung von Weichteilen, z.B. des Gehirns, wird bevorzugt die Kernspintomographie angewandt. Zur Aufnahme und zur nachträglichen Positionserfassung werden sogenannte Marker am Kopf des Patienten angebracht. Die Marker weisen eine Flüssigkeit oder ein Gel auf, welches je nach dem angewandten Tomographieverfahren für eine ausreichende Kontrastdarstellung und damit Erkennbarkeit der Marker sorgt.

Nach einem Datentransfer über ein lokales Netzwerk oder entsprechende Speichermedien werden die gescannten Aufnahmen automatisiert weiterverarbeitet, wobei das Ziel dieser Aufbereitung oder Bildbearbeitung darin besteht, eine 3D-Darstellung des Gehirns möglichst authentisch zu erreichen, um den Chirurgen bereits vor dem Eingriff in die Lage zu versetzen, die Operation zu planen und diese minimalinvasiv zu gestalten. Über Segmentierungsschritte wird durch Anwendung mathematischer Verfahren die Möglichkeit geschaffen, bestimmte anatomische Strukturen aus den Summendatensätzen herauszuarbeiten.

Für die Neuronavigation ist es dann erforderlich, eine Verbindung zwischen dem erhaltenen virtuellen Bild des Gehirns, welches sich aus den gescannten Daten erstellen läßt, und dem realen Gehirn im Operationssaal herzustellen.

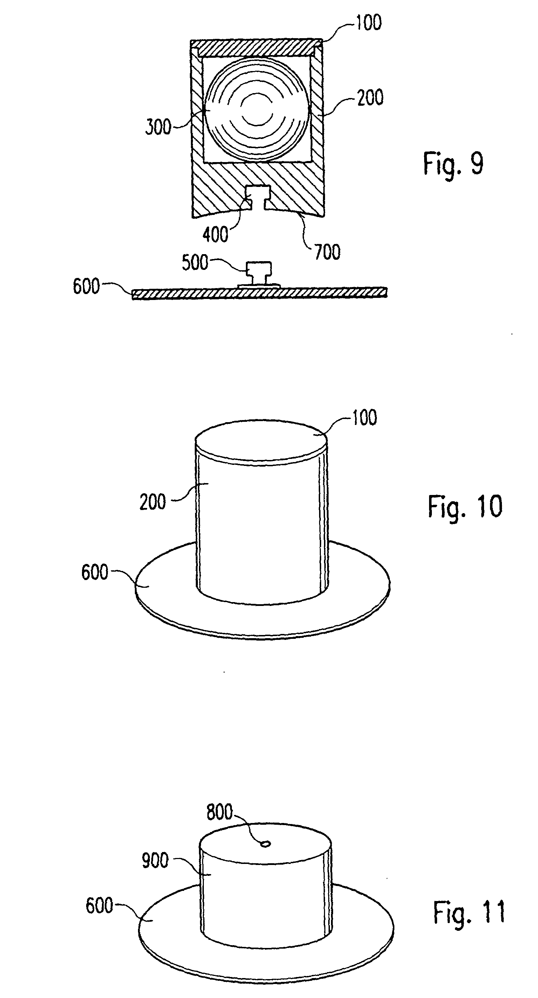

Hierfür werden eindeutige Positionen benötigt, welche sich sowohl im realen als auch im virtuellen System wiederfinden. Um derartige Positionen zu bestimmen, werden zusätzliche Marker verwendet, welche als Referenzpunkte dienen. Die Marker befinden sich in derselben Umgebung wie diejenigen, welche für die MR- oder CT-Aufnahmen Verwendung fanden. Bezüglich des Aufbaus und des Einsatzes der Marker gemäß Ausführungsbeispiel sei auf die nachfolgende Beschreibung der Fig. 9 bis 11 verwiesen.

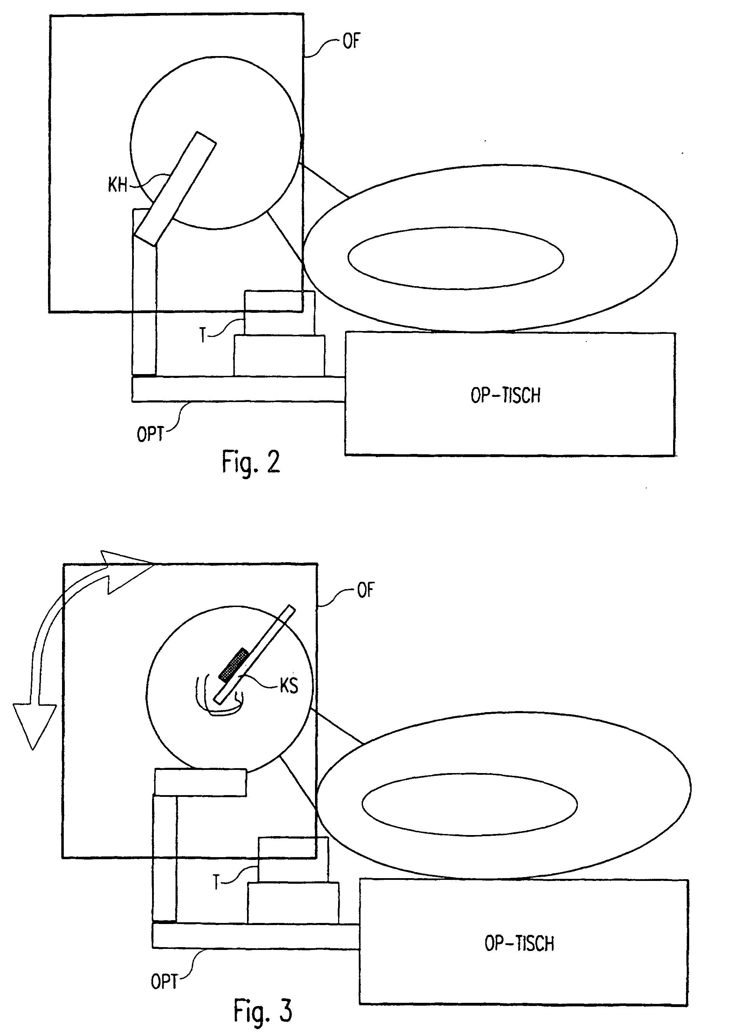

Zur Positionserfassung besitzt das Navigationssystem gemäß Ausführungsbeispiel einen magnetischen Gleichfeld-Transmitter T, welcher am bzw. mit dem Operationstisch OPT verbunden ist. Der Kopf des Patienten ist, wie in der Fig. 2 dargestellt, über eine spezielle Kopfhalterung KH fixierbar. Im Operationsfeld OF befinden sich keine den Operateur behindernden Einrichtungen der Sensorik bzw. des Navigationssystems.

Bei einem weiteren Ausführungsbeispiel, illustriert anhand der Fig. 3, ist ein weiterer Sensor, ein sogenannter Kopfsensor KS am Kopf des Patienten befestigt, um bei einer nicht starren Fixierung des Kopfes Bewegungen dieses automatisch zu erfassen, ohne daß ein manuelles Kalibrieren je nach Lageveränderung erforderlich wird. Die vom Kopfsensor erfaßten Korrekturdaten werden einem nicht gezeigten Steuerrechner zugeführt, um ein quasi dynamisches oder bezogen auf die Ursprungsänderung veränderliches Koordinatensystem zu bestimmen, ohne daß die Eigenschaften des Navigationssystems, d.h. die exakte Zuordnung von virtuellem und reellem Bild verlorengehen.

Aufgrund der festen Lagebeziehung zwischen dem Transmitter T und dem Patienten treten keine durch mechanische Erschütterungen bedingte oder auf Veränderungen oder Bewegungen des Operationstisches im Raum zurückzuführende Ungenauigkeiten bei der Positionszuordnung und der Bilddarstellung anhand der gespeicherten 3D-Datensätze auf.

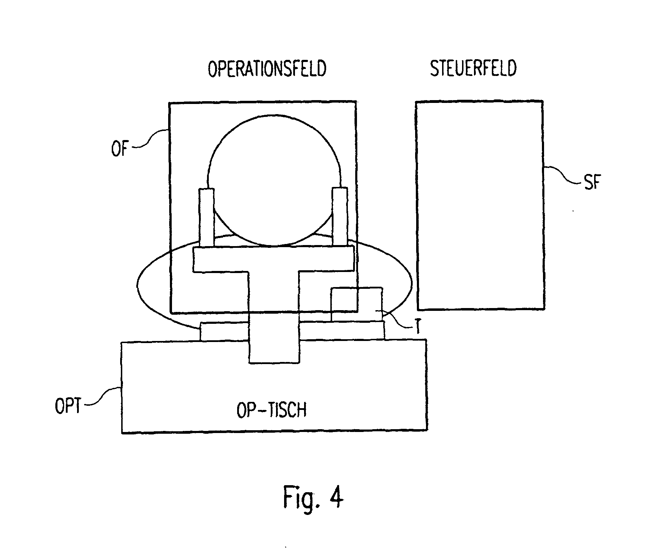

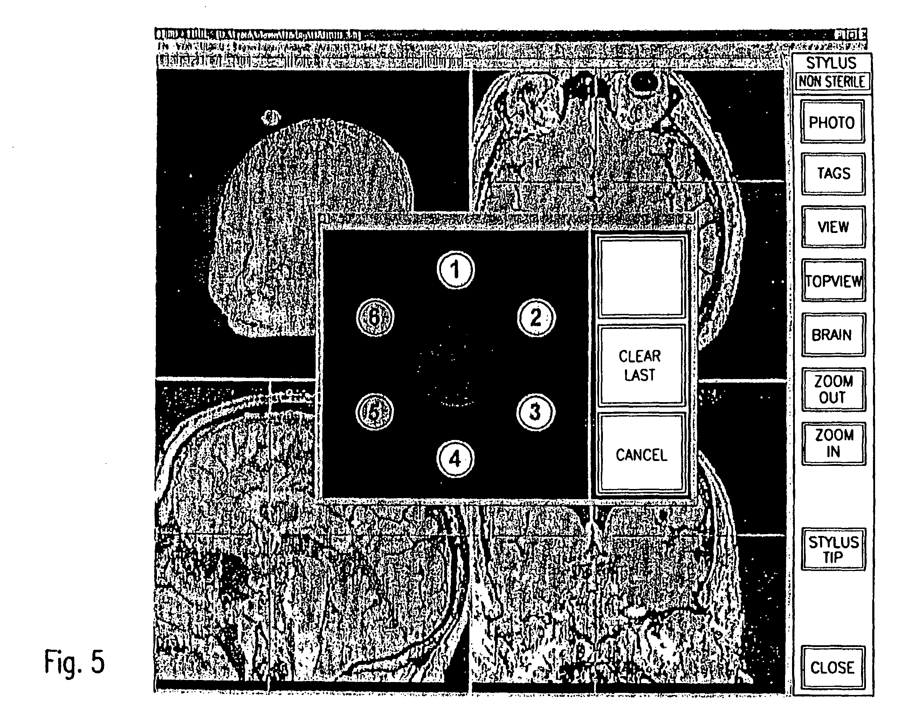

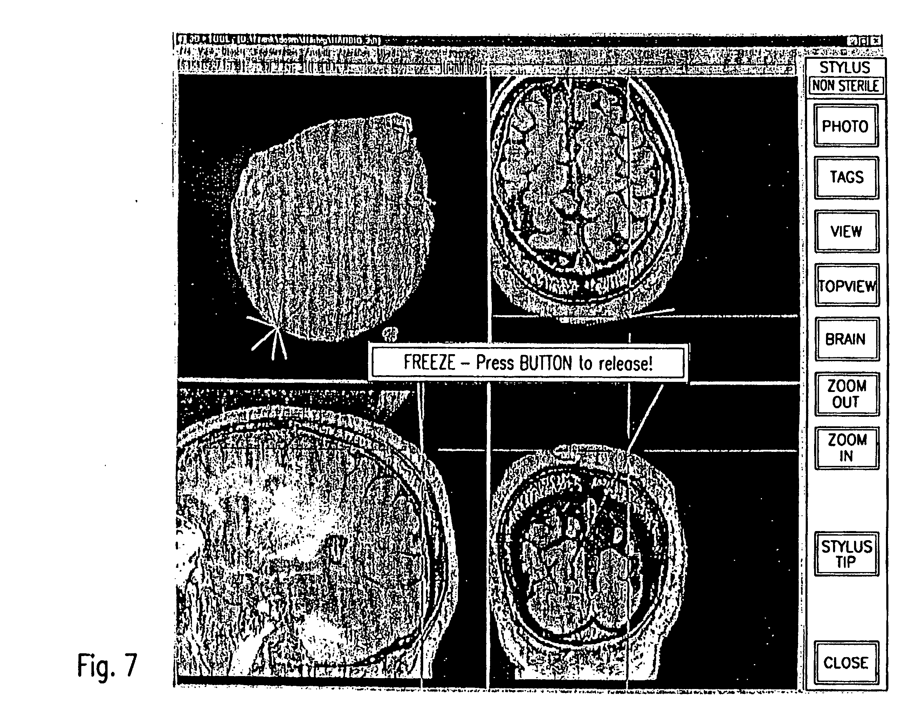

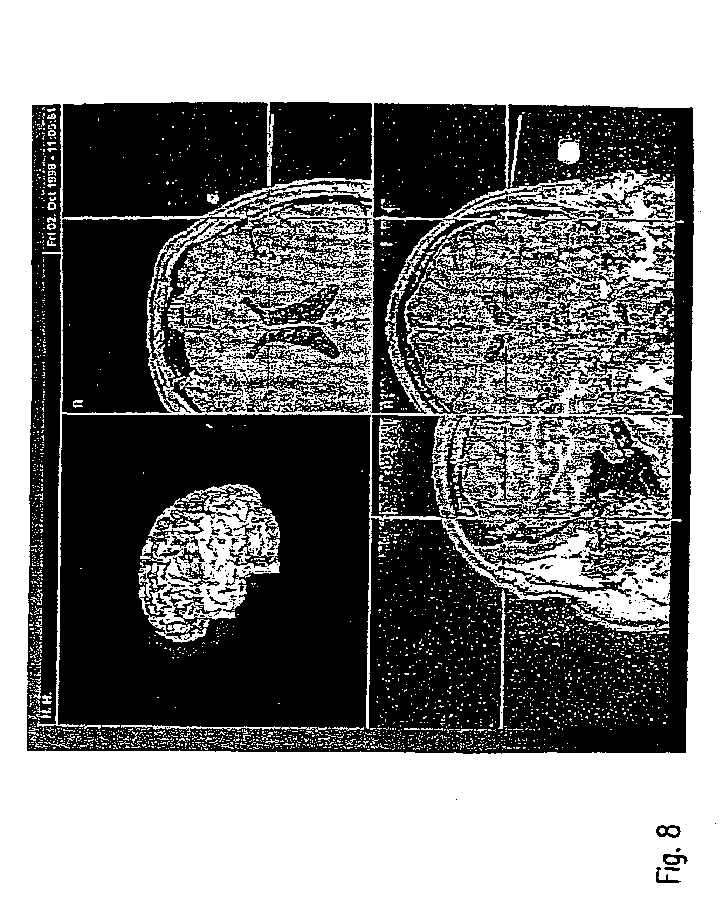

Mit Hilfe der Fig. 4 soll verdeutlicht werden, wie ohne weitere Eingabehilfsmittel allein durch Aufteilung von Operations- OF und Steuerfeld SF und Hinein- oder Herausbewegen des später beschriebenen Zeigers oder Stylus bezüglich dieser Felder ein Aktivieren der in den Fig. 5 bis 8 gezeigten Menüs und das Auslösen von Steuerbefehlen erfolgen kann.

Das entsprechende Umschalten erfolgt durch das Erkennen des Überschreitens eines räumlichen Abstands oder Grenze, in der sich der im Zeiger befindliche Sensor, der das Magnetfeld ausgesendet vom Transmitter erfaßt, befindet. Entsprechende Steuerbefehle können durch ein im Gehäuse des Zeigers befindliches elektronisches Schaltelement, z.B. Taster, ausgelöst oder bestätigt werden.

Fig. 5 zeigt eine Hardkopie des Navigationsscreens, d.h. ein Monitorbild, wobei sich der Arbeitsablauf gerade im Stadium des Prozesses der Registrierung der Marker befindet. Wie dem Monitorbild entnommen werden kann, sind z.B. bis zu sechs Marker verwendbar, so daß sich insgesamt eine hohe Reproduzierbarkeit auch dann ergibt, wenn im Laufe der Operationsvorbereitung sich ein Marker löst oder entfernt werden muß. An sich sind beliebig viele Marker verwendbar, wobei eine optimale Zahl bei 4 bis 5 liegt.

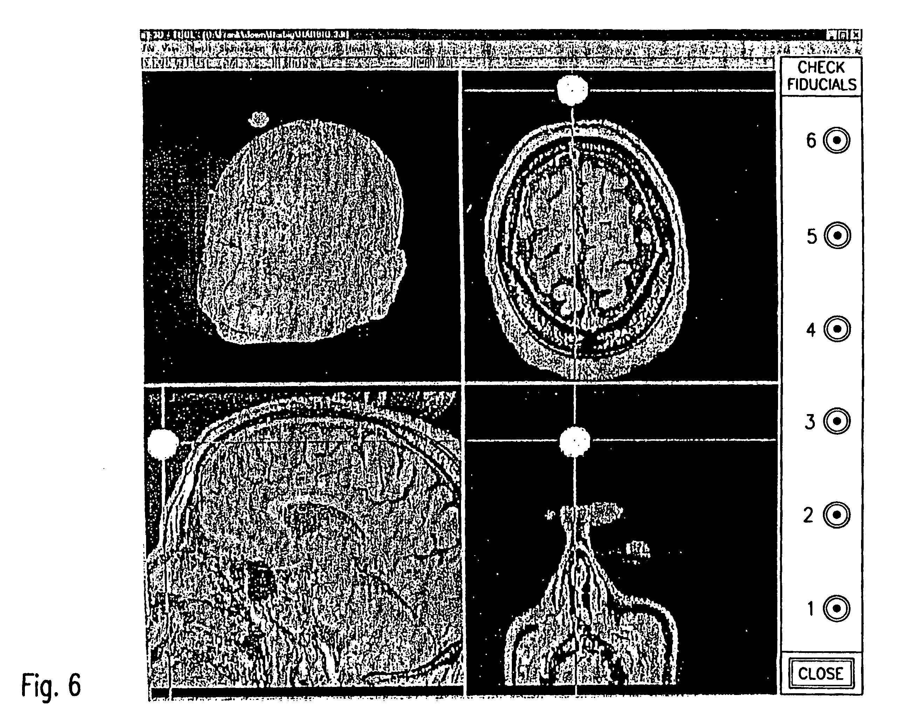

Die Fig. 6 bis 8 zeigen weitere Aufnahmen des Navigationsscreens, und zwar das sogenannte Fiducial-Management, das virtuelle Keypad zum Steuern des Navigationssystems und die Möglichkeit der intraoperativen Dokumentation zur nachfolgenden Bewertung oder Auswertung der Behandlung. Hinsichtlich des Einmeßvorgangs mit Hilfe der Einmeßkörper nach Fig. 11 sei angemerkt, daß dieser Schritt zur Herstellung einer vorgegebenen räumlichen, d.h. koordinatenseitig definierten Beziehung des Kopfes des Patienten zu seinem auf dem Monitor darstellbaren virtuellen Bild erfolgt. Hierfür werden die Einmeßhilfen respektive die Einmeßkörper auf die speziellen Träger aufgesteckt, die sich bereits auf der Haut des Patienten befinden. Die Einmeßkörper weisen, wie später erläutert, Vertiefungen auf, die mit der Tastspitze des Zeigers bzw. des Stylus in beliebiger Reihenfolge berührt werden. Das Erreichen dieser Position wird durch Betätigung des erwähnten Tasters, der sich am Zeiger befindet, bestätigt. Die Position selbst wird durch automatische Lageerfassung des im Zeiger vorhandenen Sensors vom Navigationscomputer registriert und durch das Aufleuchten einer Schaltfläche am Display angezeigt. Nach der Registrierung aller Positionen ist das Navigationssystem einsatzbereit, wobei für neurochirurgische Anwendungen eine Voreinstellung dergestalt gegeben ist, daß das 3D-Abbild des Gehirns zunächst aus der Blickperspektive des Operateurs dargestellt wird. Selbstverständlich kann mit Hilfe des Zeigers und der Funktion "View" des virtuellen Menüs diese Blickrichtung jederzeit geändert werden.

Die erwähnten Marker können vor dem Eingriff vom Kopf des Patienten entfernt werden, so daß Behinderungen ausgeschlossen sind.

Die Markierungseinrichtungen oder Fiducials für das vorgeschlagene Navigationssystem sollen nachstehend unter Bezugnahme auf die Fig. 9 bis 11 näher erläutert werden.

Die einzusetzenden Markierungseinheiten werden am entsprechenden Objekt, beispielsweise am menschlichen Schädel befestigt und gemeinsam mit den jeweiligen bildgebenden Verfahren aufgrund der vorhandenen Kontrastsubstanzen abgebildet. Davon unabhängig ist die Position dieser Markierungseinheiten zu einem späteren Zeitpunkt, nämlich im Operationssaal definierbar. Mittels einer Zusammenführung von Positionsdaten über die Lage der Markierungseinheiten auf dem bildgebenden Verfahren, d.h. in den Bilddatensätzen und in der Realität besteht dann die Möglichkeit eines Koordinatenangleichs. Dies wiederum ist die Grundlage der Zuordnung beliebiger Punkte im gegebenen Koordinatensystem, so daß eine entsprechende Navigation möglich wird.

Fig. 9 zeigt eine Querschnittsdarstellung durch die spezielle, teilbare Markierungseinrichtung. Ein am Probanden befestigbarer Träger 600 besteht bevorzugt aus einer mit einer einseitigen Klebebeschichtung versehenen Kunststofffolie. Demnach ist also der Träger 600 als flächiges Gebilde ausgeführt und besitzt vorzugsweise eine Kreisform. Der Träger ist verschieden ausführbar, z.B. mit einer großen Kleberfläche für Erwachsene und einer kleinen für Kinder, die mit jeweils unterschiedlichen Klebebeschichtungen an die verschieden empfindlichen Hauttypen angepaßt worden sind. Im Ausführungsbeispiel ist der Rastknopf aus Carbon, was den Vorteil hat, daß bei hoher Festigkeit weder eine Artefaktbildung in der Kernspintomographie noch in der Computertomographie hervorgerufen wird.

Auf der der Klebefläche abgewandten Seite des Trägers 600 ist ein hervorstehender Rastknopf 500 vorhanden. Dieser Rastknopf 500 dient der Befestigung eines zylindrischen Gehäuses 200, welches bodenseitig eine Rastaufnahme oder ein Rastgegenstück 400 besitzt. Die Bodenfläche 700 des zylindrischen Gehäuses 200 ist konkav geformt oder besitzt einen entsprechenden Rücksprung.

Durch Rastknopf 500 und Rastaufnahme 400 kann das zylindrische Gehäuse mit dem Träger 600, der bereits am Patienten fixiert ist, verbunden werden. Nach erfolgter Aufnahme, d.h. dem Tomographie-Scanning, kann der gut handhabbare, hervorstehende zylindrische Körper bzw. das zylindrische Gehäuse 200 vom Patienten entfernt und es kann dann im Operationssaal zum Einmessen der in der Fig. 11 gezeigten Einmeßkörper 900 aufgesteckt werden.

Das zylindrische Gehäuse 200 besitzt, wie in der Fig. 9 erkennbar, eine Öffnung, welche wiederum der Aufnahme einer Kugel 300 dient, welche im Inneren eine Kontrastflüssigkeit oder ein Kontrastgel für die Kernspin- bzw. Computertomographie aufnimmt.

Das zylindrische Gehäuse 200 ist mit einem durch ein Werkzeug abnehmbaren Deckel 100 verschlossen, wobei der Deckel 100 bevorzugt aus einem durchsichtigen Kunststoffmaterial besteht. Durch letztere Materialeigenschaft besteht die Möglichkeit, den Zustand der Kugel 300 respektive der dort befindlichen Markierungsflüssigkeit zu erfassen. Gleichzeitig kann die Kugel 300 mit der Markierungssubstanz gegen eine andere geeignete ausgetauscht werden. Grundsätzlich sind die Fiducials fertige Gebilde, die für die jeweiligen Aufnahmetechniken optimierte Kugeln enthalten.

Die Form der Bodenfläche 700 ermöglicht es, Krümmungen des Objekts, beispielsweise des menschlichen Schädels, auszugleichen, ohne daß sich der Sitz der Markierungseinrichtung ändert oder instabil wird. Darüber hinaus ist in dieser Bodenfläche 700 die erwähnte Ausnehmung als Rastaufnahme 400 respektive Gegenstück zum Rastknopf 500 befindlich.

Das Material des Trägers 600 mit der Klebefläche besitzt eine an die Haut angepaßte Beweglichkeit und kann der Körperoberfläche folgen, ist jedoch in sich verzerrungsstabil, so daß die zur exakten Markierung erforderliche Position des Rastknopfs 500 bevorzugt in der Mitte des Trägers 600 erhalten bleibt. Die Öffnung im zylindrischen Gehäuse 200 der Markierungseinrichtung ist so beschaffen, daß die Kugel mit der Markierungssubstanz sicher und quasi unbeweglich, d.h. in ihrer Lage stabil gehalten wird.

Fig. 2 zeigt die komplette Markierungseinrichtung, d.h. den Träger 600 mit aufgestecktem zylindrischen Gehäuse 200 inklusive Deckel 100.

Nach Durchführung der Tomographieaufnahmen wird das zylindrische Gehäuse 200 mit der die Kontrast-Markierungssubstanz aufnehmenden Kugel 300 vom Träger 600 über die lösbare Verbindung entfernt und es kann sich der Patient ungehindert bewegen. Durch die am Patienten verbleibende Trägerfolie bzw. den anhaftenden Träger 600 mit Rastknopf 500 besteht des weiteren die Möglichkeit, unter exakter Positionszuordnung präoperativ den gewünschten Einmeßvorgang durchzuführen. Für das Einmessen wird die in der Fig. 3 gezeigte Einmeßhilfe in Form eines Einmeßkörpers 900 aufgesteckt. Der Einmeßkörper stellt bevorzugt einen Vollkörper aus Kunststoff dar, dessen wesentliches Merkmal eine Vertiefung 800 im Zentrum ist, wobei diese Vertiefung 800 exakt dem Mittelpunkt bzw. dem Schwerpunkt der Kugel 300 mit der Markierungssubstanz entspricht. Auf diese Weise ist sichergestellt, daß beim Einmessen mit dem Zeiger oder Stylus der virtuelle und der reale Kugelmittelpunkt der Markierungseinrichtung zur Übereinstimmung kommen. Es ist also nach Einmessen eine exakte Überlagerung der virtuellen Bilder aus dem Navigationssystem und des realen Schädels möglich.
Nach erfolgtem Einmessen kann der Einmeßkörper, aber auch der gesamte Marker, d.h. der Träger, vom Kopf entfernt werden.

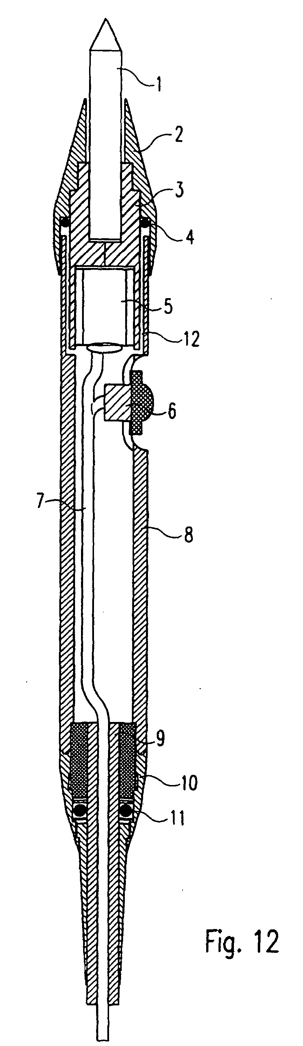

Bezüglich der Ausführung des Zeigers oder Stylus für eine Tracking-Einrichtung des vorgestellten Navigationssystems sei auf Fig. 12, welche eine Schnittdarstellung zeigt, verwiesen.

Der Zeiger weist eine Tastspitze 1 oder aber auch eine Tastspitzenaufnahme, z.B. für Einführhilfen auf. Gegenüberliegend befindet sich ein spezieller elektromagnetischer Sensor 5. Der Sensor 5 und die Tastspitze 1 sind über einen Körper 3 fest miteinander verbunden. Der Körper 3 wiederum ist bezüglich des eigentlichen langgestreckten Handstück-Gehäuses 8 nachgiebig und spannungsfrei, quasi kardanisch, gelagert.

Der Verbindungskörper 3 besteht bevorzugt aus einem verformungs- und temperaturstabilen Kunststoff oder Titan.

Im langgestreckten Gehäuse 8 befindet sich eine Öffnung zur Aufnahme eines Tasters 6. Der elektrische Ausgang des Sensors 5 ist ebenso wie der Anschluß des Tasters 6 über eine Signalleitung 7 verbunden und nach außen geführt.

Der Taster 6 ist im Handstück-Gehäuse 8 dampf- und flüssigkeitsdicht befestigt.

An dem der Tastspitze 1 gegenüberliegenden bzw. dem distalen Ende des Zeigers gegenüberliegenden Ende des Gehäuses 8 ist ein dampf- und flüssigkeitsdichter Kabelauslaß 9 befindlich, wobei der Verbindungskörper 3 und der Kabelauslaß 9 durch Überwürfe 2, 10 mechanisch lösbar mit dem Gehäuse 8 verbunden sind. Zum Gewährleisten der erforderlichen Dichtheit sind elastische Dichtelemente bevorzugt in Form von Dichtringen 4 und 11 im Bereich der Überwürfe 2 und 10 vorhanden.

Der Verbindungskörper 3 befindet sich mindestens mit seiner Sensoraufnahmeöffnung, d.h. mit dem Sensor 5 innerhalb des Gehäuses 8, wobei zwischen dem Verbindungskörper 3 und der Gehäuseinnenseite ein Ringspalt 12 ausgebildet ist.

Mit Hilfe der Tastspitze 1 wird das zu navigierende Objekt berührt, wobei die entsprechende Lage durch die Zuordnung von Sensor 5 und Tastspitze 1 und dem sich hieraus ergebenden Vektor ermittelt wird. Diese Lageposition dient dem Steuerrechner des Navigationssystems zum Selektieren derjenigen virtuellen Bilder, welche sich in quasi Blickrichtung der Tastspitze befinden.

Der Verbindungskörper 3 besteht bevorzugt aus einem verformungs- und temperaturstabilen Kunststoff oder Titan. Der Einsatz eines solchen Materials sichert die gewünschte feste Verbindung zwischen Tastspitze 1 und Sensor 3, wobei gleichzeitig über den Ringspalt 12 der mechanische Kontakt zum Gehäuse 8 auf ein Minimum reduziert ist. Verformungskräfte, die beispielsweise bei einer Dampfsterilisation auf den Zeiger, insbesondere das Gehäuse wirken, werden von der Verbundanordnung aus Spitze 1, Körper 3 und Sensor 5 ferngehalten.

Die gezeigte Ausführungsform des Stylus geht von einem drahtgebundenen Signalaustausch zum Navigationsgerät aus. Alternativ kann jedoch auf eine drahtlose Verbindung über eine an sich bekannte Telemetrieeinrichtung zurückgegriffen werden.

Als Sensor 5 wird bevorzugt ein bekannter Magnetfeldsensor verwendet. Ein derartiger Sensor dient der räumlichen Erfassung der Sensorposition in sechs Freiheitsgraden, wobei der Sensorbewegungsbereich nicht eingeschränkt ist. Der Magnetfeldsensor erfaßt seine oder die ihm zugeordnete Position der Tastspitze 1 aufgrund eines in der Navigationsumgebung vorhandenen DC-Magnetfelds durch entsprechende Feldstärkemessung in Echtzeit.

Der spezielle Sensor ist in Verbindung mit einer zugehörigen Bewertungseinrichtung und den erwähnten Transmittern in der Lage, bis zu 144 Messungen pro Sekunde bei einer Winkelauflösung von ca. 0,1° durchzuführen. Die Ausgangsdaten stehen in einem kartesischen Koordinatensystem mit Orientierungswinkeln, aber auch in Form einer Rotationsmatrix zur Verfügung.

Alles in allem gelingt es mit vorstehender Erfindung, ein Navigationssystem anzugeben, welches durch die Kombination einer speziellen Tracking-Sensorik und einer 3D-Bilddatenaufbereitung von CT- und MR-Daten hochgenau dem realen Abbild entsprechende virtuelle Darstellungen, z.B. des Gehirns eines Patienten, zu erzeugen, so daß prä- und intraoperativ die jeweilige Behandlungsoperationsstrategie optimierbar ist. Durch den Verzicht auf zusätzliche Bedieneinheiten oder eine aufwendige Steuerung ist sowohl der personelle als auch materialle Einsatz bei der Anwendung eines derartigen Navigationssystems minimal.

### Bezugszeichenliste

- OF: Operationsfeld
- SF: Steuerfeld
- KH: Kopfhalterung
- T: Transmitter
- OPT: Operationstisch
- KS: Kopfsensor
- 1: Tastspitze
- 2, 10: Überwurf
- 3: Verbindungskörper
- 4, 11: Dichtringe
- 5: Sensor
- 6: Taster
- 7: Signalleitung
- 8: Gehäuse
- 9: Kabelauslaß
- 12: Ringspalt
- 100: Deckel
- 200: zylindrisches Gehäuse
- 300: Kugel mit Markierungssubstanz
- 400: Rastaufnahme
- 500: Rastknopf
- 600: Träger
- 700: Bodenfläche
- 800: Markierungsausnehmung
- 900: Einmeßkörper

## Patentansprüche

1. Navigationssystem zur Durchführung und Unterstützung von chirurgischen Eingriffen, insbesondere auf dem Gebiet der Neurochirurgie und der Hals-Nasen-Ohrenheilkunde,
- wobei das System eine Bilddatenbank für präoperativ angefertigte Kernspinoder Computertomographie (CT oder MR)-Aufnahmen,
- einen Personalcomputer oder Steuerrechner mit Monitor zur Bilddatenverarbeitung und -darstellung,
- eine Tracking-Einrichtung zum Bestimmen der momentanen Position eines Instruments und Ableiten oder Real-time-Nachziehen von Darstellungen der Patientenanatomie anhand der in der Bilddatenbank abgelegten Aufnahmen,
- Mittel zum Extrahieren von anatomischen Strukturen aus den Rohdatensätzen der präoperativen Aufnahmen und Bereitstellen dieser Strukturen in Form von visualisierbaren 3D-Datensätzen und
- einen Transmitter zum Erzeugen eines definierten magnetischen DC-Gleichfelds in der Navigationsumgebung sowie ein Zeigernavigationsinstrument mit einem integralen Magnetfeldsensor aufweist, wobei der Magnetfeldsensor und der Gleichfeldtransmitter die Tracking-Einrichtung bilden und der Magnetfeldsensor seine oder die zugeordnete Position des Instruments durch richtungsorientierte Feldstärkemessung erfasst,
**gekennzeichnet durch**
- Mittel zur Menü-geführten Steuerung des Systems, wobei **durch** Bewegungen des Zeigernavigationsinstruments ausserhalb des Operationsfelds, aber innerhalb der Navigationsumgebung ein Aktivieren oder Deaktivieren angebotener Menüs oder Steuermaßnahmen erfolgt, wobei weiterhin der Umschaltmoment **durch** das Überschreiten eines räumlichen Abstands oder einer Grenze, in der sich der Magnetfeldsensor befindet, festgelegt ist und Steuerbefehle **durch** einen Schalter oder Taster im Instrument ausgelöst oder bestätigt werden.

2. Navigationssystem nach Anspruch 1,
**dadurch gekennzeichnet, dass** über ein Bildverarbeitungsmodul des Personalcomputers eine dreidimensionale Aufbereitung der Computertomographie und Kernspintomographie-Daten des Probanden mit der Folge durchgeführt wird, dass die derart erhaltenen Daten ein bezogen auf die Craniotomie-Erkenntnisse identisches Abbild zur Operations- und Behandlungsplanung ergeben.

3. Navigationssystem nach Anspruch 2,
**dadurch gekennzeichnet, dass** mittels des Bildverarbeitungsmoduls anatomische Strukturen eigenschafts- oder umfeldorientiert aus den Summendatensätzen selektierbar sind und auf der Basis einer vorgebbaren Segmentierungsstrategie diskrete Datensätze erstellt werden.

4. Navigationssystem nach einem der vorangegangenen Ansprüche,
**dadurch gekennzeichnet, dass** der Transmitter zum Erzeugen des Magnetfelds der Tracking-Einrichtung am Operationstisch oder einer dort vorgesehenen Kopfauflage, jedoch ausserhalb des Operationsfelds angeordnet ist, wodurch sich eine feste, reproduzierbare Lagebeziehung zwischen dem zu navigierenden Organ des Patienten und Transmitter unabhängig von der Lage des Operationstisches im Raum ergibt.

5. Navigationssystem nach einem der Ansprüche 2 bis 4,
**dadurch gekennzeichnet, dass** der Magnetfeldsensor Signale zum Ableiten von Position und/oder Bewegungsrichtung des Zeigernavigationsinstruments anhand des definierten magnetischen Gleichfelds und dessen Feldorientierung bereitstellt, wobei diese Signale sowohl auf dem Monitor darstellbar sind als auch zur Steuerung der Nachlade- und Aktualisierungsvorgänge des Bildbearbeitungsmoduls dienen.

6. Navigationssystem nach einem der vorangegangenen Ansprüche,
**dadurch gekennzeichnet, dass** ein weiterer Magnetfeldsensor vorgesehen ist, wobei dieser Sensor am Probanden, bevorzugt am Kopf, befestigbar ist, um Lage- und Positionsveränderungen, bezogen auf den Gleichfeld- Transmitter zu erfassen.

7. Navigationssystem nach Anspruch 6,
**dadurch gekennzeichnet, dass** die Ausgangssignale des zweiten Magnetfeldsensors als Korrekturdaten dem Steuerrechner zugeführt werden, um ein quasi dynamisches oder bezogen auf die Ursprungsänderung veränderliches Koordinatensystem zu bestimmen.

8. Navigationssystem nach einem der vorangehenden Ansprüche, **gekennzeichnet durch** eine Markierungseinrichtung oder ein Fiducial zur Positionserfassung der Lage eines Probanden und Koordinatenzuordnung bei chirurgischen Eingriffen mit Unterstützung des Navigationssystems, umfassend einen am Probanden mittels Klebefläche befestigbaren flächigen Träger sowie eine in einem vom Träger trennbaren Behältnis befindliche Kontrast-Markierungssubstanz, wobei
- der Träger als flächiges, mindestens teilflexibles Gebilde mit einem im wesentlichen zentrischen, auf der der Klebefläche abgewandten Seite hervorstehenden Rastknopf ausgebildet ist,
- die Kontrast-Markierungssubstanz von einem im Wesentlichen hohlzylindrischen Gehäuse aufgenommen ist, wobei der Gehäuseboden eine konkave Form oder einen Rücksprung aufweist und am Gehäuseboden ein Rastrücksprung vorgesehen ist, wobei **durch** die Gehäusebodenform Objektkrümmungen ausgleichbar sind.

9. Navigationssystem nach Anspruch 8,
**dadurch gekennzeichnet, dass** das zylindrische Gehäuse einen abnehmbaren Gehäusedeckel, vorzugsweise aus einem durchsichtigen Kunststoff aufweist, so dass die Markierungssubstanz kontrollier- und austauschbar ist.

10. Navigationssystem nach Anspruch 8 oder 9,
**dadurch gekennzeichnet, dass** die Markierungssubstanz für die Kemspintomographie eine Flüssigkeit oder ein Gel ist, welche bzw. welches sich in einem Kugel- oder geschlossenen Zylindergefäss befindet, wobei der Aussendurchmesser der Kugel oder des Zylinders im Wesentlichen dem Innendurchmesser des zylindrischen Gehäuses mit Rastaufnahme entspricht.

11. Navigationssystem nach einem der Ansprüche 8 bis 10,
**dadurch gekennzeichnet, dass** ein geschlossener, in seinen Abmessungen und seiner bodenseitigen Ausbildung dem zylindrischen Gehäuse entsprechender Einmesskörper vorgesehen ist, wobei der Einmesskörper deckelseitig eine Markierungsausnehmung hat, welche sich in einer Position befindet, die gleich dem Mittel- oder Schwerpunkt der Kontrast-Markierungssubstanz bzw. des Kugel- oder Zylindergefässes ist.

12. Navigationssystem nach einem der Ansprüche 8 bis 12,
**dadurch gekennzeichnet, dass** das zylindrische Gehäuse und der Einmesskörper aus Kunststoffmaterial bestehen, wobei der Träger bevorzugt eine einseitige klebebeschichtete Folie umfasst.

13. Navigationssystem nach einem der vorangehenden Ansprüche, **gekennzeichnet durch** einen Zeiger für eine Tracking-Einrichtung des Navigationssystems zur Durchführung und Unterstützung von chirurgischen Eingriffen mit einem in einem langgestreckten Handstück-Gehäuse angeordneten gekapselten Sensor sowie einer aus dem Gehäuse teilweise hervorstehenden Tastspitze, wobei der gekapselte Sensor mit der Tastspitze oder einer Tastspitzen- bzw. Einführhilfenaufnahme mittels eines im Wesentlichen gegenüberliegende Öffnungen aufweisenden Körpers starr verbunden ist, wobei der Körper mit jeweils in den Öffnungen befindlichem Sensor und Tastspitze bezüglich des Handstück- Gehäuses nachgiebig, spannungsfrei, quasi kardanisch gelagert ist und der Verbindungskörper aus einem verformungs- und temperaturstabilen Kunststoff oder Titan besteht.

14. Navigationssystem nach Anspruch 13,
**dadurch gekennzeichnet, dass** in der Wandung des Handstück-Gehäuses ein Signaltaster dampf- und flüssigkeitsdicht angeordnet ist.

15. Navigationssystem nach einem der Ansprüche 13 oder 14,
**dadurch gekennzeichnet, dass** an dem der Tastspitze gegenüberliegenden bzw. dem distalen Ende des Gehäuses gegenüberliegend ein dampf- und flüssigkeitsdichter Kabelauslass befindlich ist, wobei der Verbindungskörper und der Kabelauslass durch Überwürfe mit dem Gehäuse verbunden sind.

16. Navigationssystem nach einem der Ansprüche 13 bis 15,
**dadurch gekennzeichnet, dass** der Verbindungskörper mindestens mit seiner Sensoraufnahmeöffnung sich innerhalb des Gehäuses befindet, wobei zwischen dem Verbindungskörper und der Gehäuseinnenseite ein Ringspalt ausgebildet und zur Abdichtung zwischen Verbindungskörper und Gehäuse oder Überwurf und Gehäuse mindestens ein elastischer Dichtring vorgesehen ist.

## Claims

1. A navigation system for performing and assisting surgical interventions, in particular in the field of neurosurgery and ear, nose, throat therapeutics,
- with the system comprising an image data base for pre-operatively made nuclear spin resonance tomography or computed tomography (MR or CT) pictures,
- a personal computer or control computer with monitor for image data processing and display,
- a tracking device for determining the current position of an instrument and deriving or real time trailing of illustrations of the patient's anatomy by means of the pictures stored in the image data base,
- means for extracting anatomic structures from the raw data records of the pre-operative pictures and making available these structures in the form of visualisable 3D data records, and
- a transmitter for generating a defined DC magnetic field in the navigation environment as well as a pointer navigation instrument with an integral magnetic field sensor, with the magnetic field sensor and the DC magnetic field transmitter forming the tracking device and the magnetic field sensor detecting its own position or the associated position of the instrument through direction-oriented field strength measurement,
**characterised by**
- means for the menu-driven control of the system, with the activation or deactivation of offered menus of control activities being effected through movements of the pointer navigation instrument outside the operation field, but within the navigation environment, further with the switch-over moment being established by exceeding a spatial distance or a boundary within which the magnetic field sensor is located, and control commands being initiated or acknowledged by a switch or button in the instrument.

2. The navigation system according to Claim 1,
**characterised in that** a three-dimensional editing of the computed tomography and the nuclear spin resonance tomography data of the test person is carried out via an image processing module of the personal computer with the result that the data obtained in this manner yield an identical picture with reference to the craniotomy findings for the operation and treatment planning.

3. The navigation system according to Claim 2,
**characterised in that** anatomic structures are selectable by means of the image processing module in a property or environment oriented manner from the aggregate data records and discrete data records are generated on the basis of a segmenting strategy which may be pre-specified.

4. The navigation system according to one of the previous claims,
**characterised in that** the transmitter for the generation of the magnetic field of the tracking device is arranged at the operating table or a head rest provided thereon, but outside the operation field, which results in a fixed reproducible positional relationship between the patient's organ to be navigated and the transmitter, irrespective of the spatial position of the operating table.

5. The navigation system according to one of Claims 2 to 4,
**characterised in that** the magnetic field sensor provides signals for deriving the position and/or direction of movement of the pointer navigation instrument on the basis of the defined DC magnetic field and its field orientation, with these signals being both capable of being displayed on the monitor and serving for the control of the reloading and updating operations of the image processing module.

6. The navigation system according to one of the previous claims,
**characterised in that** a further magnetic field sensor is provided which may be attached at the test person, preferably at his or her head, in order to detect locational and positional changes relative to the DC magnetic field transmitter.

7. The navigation system according to Claim 6,
**characterised in that** the output signals of the second magnetic fled sensor are supplied as correction data to the control computer in order to determine a quasi dynamic or variable coordinate system relative to the change of origin.

8. The navigation system according to one of the previous claims, **characterised by** a marking device or a fiducial for position detection of the location of a test person and coordinate assignment in surgical interventions with the assistance of the navigation system, comprising a plane carrier which may be attached at the test person by means of an adhesive surface, as well as a contrast marking substance which is held in a reservoir adapted to be removable from the carrier, with
- the carrier being formed as a plane at least partially flexible object with an essentially central detent button which protrudes from the side facing away from the adhesive surface,
- the contrast marking substances being accommodated in an essentially hollow cylindrical housing, with the housing bottom comprising a concave shape or a recess and with a detent recess being provided on the housing bottom, with the shape of the housing bottom compensating for object curvatures.

9. The navigation system according to Claim 8,
**characterised in that** the cylindrical housing comprises a removable housing lid, preferably made of a transparent plastic material, so that the marking substance may be monitored and exchanged.

10. The navigation system according to Claim 8 or 9,
**characterised in that** the marking substance for the nuclear spin resonance tomography is a liquid or a gel which is held in a spherical or closed cylindrical vessel, with the outer diameter of the sphere or the cylinder essentially corresponding to the inner diameter of the cylindrical housing with detent recess.

11. The navigation system according to one of Claims 8 to 10,
**characterised in that** a closed fitting body is provided whose dimensions and bottom side shape correspond to the cylindrical housing, with the fitting body having a lid side marking recess which is located in a position which is identical with the centre or centre of gravity of the contrast marking substance or the spherical or cylindrical vessel, respectively.

12. The navigation system according to one of Claims 8 to 12,
**characterised in that** the cylindrical housing and the fitting body consist of plastic material, with the carrier preferably comprising a single-sided adhesive coated sheet.

13. The navigation system according to one of the previous claims, **characterised by** a pointer for a tracking device of the navigation system for performing and assisting surgical interventions with a sensor encapsulated in a longitudinal hand-held housing as well as a probe tip partially protruding from the housing, with the encapsulated sensor being rigidly connected with the probe tip or a probe tip or insertion assisting receptacle by means of a body comprising essentially opposite openings, with the body with the sensor and probe tip located in the respective openings being supported resiliently, stress-free, quasi cardanically, and the connecting body consisting of a deformation resistant and temperature stable plastic material or titanium.

14. The navigation system according to Claim 13,
**characterised in that** a signal button is arranged in a vapour und fluid-tight manner in the wall of the hand-held housing.

15. The navigation system according to one of Claims 13 or 14,
**characterised in that** a vapour and fluid tight cable outlet is provided at the end of the housing opposite the probe tip or opposite the distal end of the housing, respectively, with the connecting body and the cable outlet being connected with the housing by means of coupling means.

16. The navigation system according to one of Claims 13 to 15,
**characterised in that** the connecting body, at least with its sensor receptacle opening, is located inside the housing, with an annular gap being formed between the connecting body and the housing inner side, and at least one elastic sealing ring being provided for sealing purposes between the connecting body and the housing or the coupling means and the housing.

## Revendications

1. Système de navigation pour réaliser et assister des interventions chirurgicales, en particulier dans le domaine de la neurochirurgie, et l'oto-rhino-laryngologie,
- dans lequel le système présente une base de données d'images pour des prises de vues réalisées de façon pré-opératoire, en imagerie par résonance magnétique nucléaire, ou par tomodensitométrie (IRM ou TDM),
- un ordinateur personnel, un calculateur de commande, avec moniteur pour le traitement et la représentation des données d'images,
- un dispositif de poursuite pour déterminer la position momentanée d'un instrument, et pour déduire ou retracer en temps réel, des représentations de l'anatomie du patient à l'aide des prises de vues déposées dans la base de données d'images,
- des moyens pour extraire des structures anatomiques provenant des enregistrements de données brutes des prises de vues pré-opératoires et la préparation de ces structures sous forme d'enregistrements de données 3D visualisables, et
- un transmetteur pour produire un champ magnétique continu défini dans l'environnement de navigation, ainsi qu'un instrument de navigation à indicateur ayant un détecteur intégral de champ magnétique, le détecteur de champ magnétique et le transmetteur de champ continu formant le dispositif de poursuite, et le détecteur de champ magnétique détecte sa position ou la position associée de l'instrument, au moyen d'une mesure d'intensité de champ, orientée en direction,
**caractérisé par**
- des moyens pour la commande du système, pilotée par un menu, une activation ou une désactivation des menus proposés ou des mesures de commande se réalisant par des mouvements de l'instrument de navigation à indicateur en dehors du champ opératoire, mais à l'intérieur de l'environnement de navigation, le moment de commutation étant de plus déterminé par le dépassement d'une distance spatiale ou d'une frontière, dans laquelle se trouve le détecteur de champ magnétique, et les instructions de commande étant déclenchées ou confirmées par un commutateur ou bouton poussoir dans l'instrument.

2. Système de navigation selon la revendication 1,
**caractérisé en ce que**, par l'intermédiaire d'un module de traitement d'images de l'ordinateur personnel, une préparation tridimensionnelle des données de tomodensitométrie et d'imagerie de résonance magnétique nucléaire du sujet est réalisée, avec pour conséquence que les données obtenues de ce type donnent une image identique par rapport aux connaissances de la craniotomie pour la planification des interventions et des traitements.

3. Système de navigation selon la revendication 2,
**caractérisé en ce que** des structures anatomiques peuvent être sélectionnées d'une façon orientée sur les propriétés ou l'environnement, à partir des enregistrements cumulés de données, au moyen du module de traitement d'images, et des enregistrements discrets de données sont produits sur la base d'une stratégie de segmentation pouvant être prédéterminée.

4. Système de navigation selon l'une quelconque des revendications précédentes,
**caractérisé en ce que** le transmetteur pour produire le champ magnétique du dispositif de poursuite est disposé sur la table d'opération ou sur un repose-tête qui y est prévu, toutefois en dehors du champ opératoire, d'où résulte une relation d'emplacement fixe reproductible entre l'organe du patient à explorer et le transmetteur, de façon indépendante de l'emplacement de la table d'opération dans l'espace.

5. Système de navigation selon l'une quelconque des revendications 2 à 4,
**caractérisé en ce que** le détecteur de champ magnétique prépare des signaux pour déduire la position et/ou la direction de mouvement de l'instrument de navigation à indicateur à l'aide du champ magnétique continu défini et son orientation de champ, ces signaux pouvant non seulement être présentés sur le moniteur mais encore servant à commander les processus de rechargement et d'actualisation du module de traitement d'images.

6. Système de navigation selon l'une quelconque des revendications précédentes,
**caractérisé en ce qu'**un détecteur de champ magnétique supplémentaire est prévu, ce détecteur pouvant être fixé sur le sujet, de préférence au niveau de la tête, afin de détecter les changements d'orientation et de position, par rapport au transmetteur de champ continu.

7. Système de navigation selon la revendication 6,
**caractérisé en ce que** les signaux de sortie du deuxième détecteur de champ magnétique sont introduits dans le calculateur de commande sous la forme de données de correction, afin de déterminer un système de coordonnées quasi-dynamique ou variable par rapport à la modification d'origine.

8. Système de navigation selon l'une quelconque des revendications précédentes,
**caractérisé par** un dispositif de marquage ou un trait de repère pour détecter la position de l'emplacement d'un sujet et l'association de coordonnées lors d'interventions chirurgicales avec l'assistance du système de navigation, comprenant un support plan pouvant être fixé au sujet au moyen d'une surface adhésive, ainsi qu'une substance de marquage pour le contraste, se trouvant dans un récipient séparable du support,
- le support étant conçu sous la forme d'une structure plane, au moins partiellement souple, ayant un bouton poussoir essentiellement centré faisant saillie du côté faisant dos à la surface adhésive,
- la substance de marquage pour le contraste étant reçue par un boîtier essentiellement à cylindre creux, le fond du récipient présentant une forme concave ou un retrait, et un retrait à crans étant prévu sur le fond du boîtier, les courbures d'objets étant compensables par la forme du fond du boîtier.

9. Système de navigation selon la revendication 8,
**caractérisé en ce que** le boîtier cylindrique présente un couvercle de boîtier amovible, de préférence constitué d'une matière plastique transparente, de sorte à pouvoir contrôler et remplacer la substance de marquage.

10. Système de navigation selon la revendication 8 ou 9,
**caractérisé en ce que**, la substance de marquage pour l'imagerie par résonance magnétique nucléaire est un liquide ou un gel, lequel se trouve dans un récipient sphérique ou cylindrique fermé, le diamètre extérieur de la sphère ou du cylindre correspondant essentiellement au diamètre intérieur du boîtier cylindrique avec réceptacle à crans.

11. Système de navigation selon l'une quelconque des revendications 8 à 10,
**caractérisé en ce qu'**un corps étalon fermé est prévu qui, dans ses dimensions et sa forme du côté du fond, correspond au boîtier cylindrique, le corps étalon ayant du côté du couvercle un évidement de marquage, lequel se trouve dans une position qui est égale à celle du centre ou centre de gravité de la substance de marquage pour le contraste, ou du récipient sphérique ou cylindrique.

12. Système de navigation selon l'une quelconque des revendications 8 à 12,
**caractérisé en ce que**, le boîtier cylindrique et le corps étalon sont constitués de matière plastique, le support comprenant de préférence une feuille revêtue d'un adhésif d'un seul côté.

13. Système de navigation selon l'une quelconque des revendications précédentes,
**caractérisé par** un indicateur destiné à un dispositif de poursuite du système de navigation, pour la réalisation et l'assistance d'interventions chirurgicales avec un détecteur encapsulé disposé dans un boîtier de pièce à main de longue extension, ainsi que par une pointe de palpeur faisant partiellement saillie du boîtier, le détecteur encapsulé étant lié rigidement à la pointe de palpeur ou à un réceptacle de pointe de palpeur ou d'aide à l'insertion, au moyen d'un corps présentant des ouvertures essentiellement opposées, le corps étant monté de façon élastique, sans contrainte, quasiment à la façon d'un cardan, par rapport au boîtier de la pièce à main, avec un détecteur et une pointe de palpeur se trouvant respectivement dans les ouvertures, et le corps de raccordement étant constitué d'une matière plastique stable à la déformation et à la température, ou de titane.

14. Système de navigation selon la revendication 13,
**caractérisé en ce qu'**un palpeur de signal est disposé de façon étanche aux vapeurs et aux liquides, dans la paroi du boîtier de la pièce à main.

15. Système de navigation selon l'une quelconque des revendications 13 ou 14,
**caractérisé en ce qu'**une sortie de câble étanche aux vapeurs et aux liquides, se trouve sur l'extrémité opposée à la pointe palpeuse, ou bien à l'opposé sur l'extrémité distale du boîtier, le corps de raccordement et la sortie de câble étant reliés par des attaches au boîtier.

16. Système de navigation selon l'une quelconque des revendications 13 à 15,
**caractérisé en ce que** le corps de raccordement au moins avec son ouverture de réceptacle de détecteur, se trouve à l'intérieur du boîtier, un espace annulaire étant formé entre le corps de raccordement et le côté intérieur du boîtier, et au moins une bague d'étanchéité élastique étant prévue pour l'étanchéité entre le corps de raccordement et le boîtier, ou l'attache et le boîtier.
